# EUROPEAN PATENT APPLICATION

(11) **EP 3 888 700 A1**
(43) Date of publication of application: **06.10.2021**
(21) Application number: 20179815.4
(22) Date of filing: 12.06.2020
(51) Int. Cl.: A61L 2/12, A61L 9/18, A47B 97/00, A47G 27/00, A47K 13/30, B66B 31/00, E03D 9/00, E05B 1/00, H04M 1/17

(54) **APPARATUS FOR VIRUS INACTIVATION**

(30) Priority: 02.04.2020 TR 202005289; 19.05.2020 EP 20175494; 02.06.2020 WO PCT/EP2020/065150
(71) Applicant: Smarte Teknoloji Ve Enerji San. Tic. A.S., 34467 Sariyer/Istanbul (TR); PLUSSMARTE DANMARK APS, 1220 København K (DK); Ballikaya, Melih, 34467 Istanbul (TR)
(72) Inventor: BALLIKAYA, Melih, 34467 Istanbul (TR)
(74) Representative: Sevinç, Erkan

(57) **Abstract**

The present invention proposes an apparatus (1) which includes one or more microwave antenna(s) (11) arranged for emitting a microwave frequency for inciting such mechanical damage in viruses. The microwave antenna(s) (11) can be arranged for emitting a frequency within the range between 1 GHz and 20 GHz; more preferably within the range between 8.0 GHz and 9.5 GHz, even more preferably between 8.2 GHz and 8.8 GHz, even more preferably between 8.3 GHz and 8.5 GHz.

## Description

### Technical field of the invention

The present invention relates to an apparatus for inactivating viruses in its surroundings. In particular, the present invention relates to an apparatus which is arranged for inactivation of viruses in its surroundings using microwave.

### Background of the Invention

Disinfection by inactivation of viruses mainly includes the use of ultraviolet (UV) radiation, or chemical agents such as oxidants.

For inactivation of viruses with the principles of chemistry, chemical agents inevitably contaminate the surfaces to be disinfected, along with any fluid (such as ambient air) in contact with said surfaces. In the cases where the fluid is air or water to be directly contacted with mammals such as humans, the chemical agents should be separated from the fluid for elimination of possible harm. Such separation process brings extra and unmanageable costs. Disinfection by chemical agents take a high amount of time (e.g. around 150 minutes), and cannot be considered to be completed without necessitating such long residence time. Hence, chemical means are not sufficiently suitable for inactivating viruses in a short time.

On the other hand, UV radiation itself is normally insufficient for an effective and swift inactivation of viruses in surroundings of an UV-based disinfection device, unless a tremendous amount of energy is consumed in the process. When air is subjected to UV radiation, ozone is formed, which is harmful to living bodies. Furthermore, when exerted directly, UV radiation is not only harmful to human skin and corneas; but also causes degradation in items such as polymeric materials, pigments/dyestuffs used in colouring the items and surface coatings.

WO 2004/069288 A2 discloses the use microwaves as an alternative to UV radiation in sterilization of air. Air is passed through a wire mesh of a sterilization chamber. A vapour or a gas is adhered onto microorganisms to form a complex structure, and then said complex structure is subjected to high energy microwaves or UV radiation to cause a high electrostatic load on the complex structure, resulting in disinfection. Apparently, heating of the complex structure is also an important part of the related disinfection process.

US 2004/ 120 845 A1 relates to removal of pathogens in air circulated by HVAC systems. The system employs an ozone generator for preparing ozone to be mixed with water to obtain an oxidizing chemical agent. UV radiation is used in the formation of ozone from air oxygen. Microwaves are discussed as an alternative to UV radiation. Accordingly, said publication uses microwaves in production of ozone as a step in process for obtaining the oxidizing chemical agent.

It is assumed that in an indoors space, airborne viruses endure for days. In the case where the indoors space is air conditioned, the viruses are easily distributed all around the space, which aggravates a spread. Thus, in the process of normalization after a pandemic such as COVID-19, use of air conditioning systems in public indoor spaces (such as restaurants, workspaces or shopping malls) must be avoided, because air streaming from the air conditioning systems expedites the spread of the infection. Therefore, all of the advantages related to air conditioning shall remain unavailable until the current pandemic is completely over.

Hence, inactivation of viruses in a swift and harmless way is still an unsolved concern.

### Objects of the Invention

The primary object of the present invention is to overcome the drawbacks outlined above.

Another object of the present invention is to propose an apparatus to be used in inactivation of viruses in its surroundings.

A further object of the present invention is to propose such apparatus which is also easy to produce with low cost, which is easy to operate, which has long service life and easy to maintain by having a simple structure.

A further object according to the present invention is to propose such apparatus, which is further arranged for data communication.

### Summary of the Invention

The apparatus proposed in the present application has a simple structure, which is easy to produce with low cost. The simple structure is also durable for a long service life, and easy to maintain. The apparatus does not consume high amounts of energy when in use, and is therefore economically advantageous in terms of operational costs. The virus inactivation process does not result in contamination with harmful chemicals such as organic disinfectants or ozone, nor causes health or economic damage.

The apparatus according to the present invention includes a microwave antenna arranged for provision of radio frequency to inactivate one or more virus species. The apparatus can be further arranged for data transfer, such as that within the context of IoT (i.e. internet of things). The apparatus can be further arranged for transmitting such data over the microwave antenna; i.e. by embedding the data onto the waves to be transmitted by the microwave antenna. The apparatus can be integrated to a fixture for initiating virus inactivation based on whether the fixture is in use.

In other words, the present invention relates to an apparatus which includes one or more microwave antenna(s) arranged for emitting a microwave frequency for inciting such mechanical damage in viruses.

Said one or more microwave antenna(s) are preferably arranged for emitting a frequency within the range between 1 GHz and 20 GHz.

Said one or more microwave antenna(s) are more preferably arranged for emitting a frequency within the range between 8.0 GHz and 9.5 GHz, even more preferably between 8.2 GHz and 8.8 GHz, even more preferably between 8.3 GHz and 8.5 GHz.

The apparatus can further comprise one or more means for communication.

The means for communication can be arranged for triggering an initiation or an increment of a power consumption of the one or more microwave antenna(s).

The apparatus can be arranged for being brought into communication with one or more sensor(s); and can comprise analog/digital connections for the arrangement of the communication between the sensor(s) and the apparatus; and can be pre-programmed to initiate and execute the virus inactivation in accordance with data/signals from the sensor(s).

The apparatus can be integrated as a part of a fixture selected from seat handle, arm rest, bar, grab handle, lighting fixture and toilet seat.

In a further approach, the apparatus can be integrated as a part of a fixture selected from seat handle, arm rest, bar, grab handle, lighting fixture and toilet seat. In such case, said one or more sensor(s) can be arranged to generate a value related to whether the fixture is in use. Here, the apparatus can be arranged to generate and transfer data related to information based on one or more of said values to a coordinating means for coordination of virus inactivation in a facility where the fixture is placed, e.g. via a mesh network or via broadcasting by embedding the data onto microwaves generated by the microwave antenna(s). Such fixture can preferably be a public transportation vehicle fixture, more preferably an airplane fixture. As an alternative, such fixture can be for being used in a real estate property, more preferably in a public service facility such as a building, e.g. in a waiting room.

An exemplary embodiment of the apparatus can be integrated as a part of a fixture which is in the form of a box, such that the one or more microwave antenna(s) are arranged for virus inactivation directed to an inner cavity of the box. A further, exemplary embodiment of the apparatus can be integrated as a part of a small home appliance or white good, wherein the one or more microwave antenna(s) are preferably in the form of an outer shell of said home appliance or white good. A further, exemplary embodiment of the apparatus can be integrated to a conveyor, such that the one or more microwave antenna(s) are arranged for covering goods to be conveyed by said conveyor. A further, exemplary embodiment of the apparatus can be integrated to a portable hand-held device, which is preferably a communication device such as mobile phone, which is even more preferably further arranged for generating communication signals embedded on the microwaves. A further, exemplary embodiment of the apparatus can be integrated to an elevator.

In an embodiment of the apparatus which is above described as being arranged for being brought into communication with one or more sensor(s); and which can comprise analog/digital connections for the arrangement of the communication between the sensor(s) and the apparatus; and which can be pre-programmed to initiate and execute the virus inactivation in accordance with data/signals from the sensor(s):
- the apparatus can be integrated as a part of a fixture, and said one or more sensor(s) can include one or more pressure sensor(s) for detecting a change in values of weight exerted onto a top surface of the fixture when, the fixture is in use, and/or a temperature sensor for detecting thermal values on the top surface. More preferably the apparatus is arranged to generate and transfer data related to information based on one or more of said values to a coordinating means for coordination of virus inactivation in a facility where the fixture is placed e.g. via a mesh network or via broadcasting, by embedding the data onto microwaves generated by the microwave antenna(s). For instance, such the fixture can be selected from seat, chair, sofa, bed, desk, carpet, rug and toilet seat.

### Brief Description of the Drawings

The appended drawings, brief description of which is provided below, are given solely for the purpose of exemplifying embodiments according to the present invention.
Fig.1 shows a schematic perspective view of an apparatus according to the present invention.
Fig.2 shows a schematic perspective view of an exemplary apparatus according to the present invention, further provided with a means for communication.
Fig.3 shows a schematic perspective view of an exemplary apparatus according to the present invention, further provided with a sensor.
Fig.4 shows a schematic perspective view of an exemplary apparatus according to the present invention, integrated to a fixture. This drawing refers to an exemplary case where the apparatus is integrated at a top surface of the fixture. In order to clarify the term "top", the gravity vector when the apparatus is in use, is represented with "g".
Fig.5 shows a schematic perspective view of an exemplary apparatus according to the present invention, integrated to a box.
Fig.6 shows an exemplary depiction of a small appliance or white good provided with the apparatus (1) according to the present invention. In the exemplary case shown in this drawing, a side surface is arranged to function as a microwave antenna (11) to broadcast virus inactivating microwaves.
Fig.7 shows a schematic perspective view of an exemplary apparatus according to the present invention, integrated to a conveyor.

### Detailed Description of the Invention

Hereinafter, preferred embodiments of the present invention will be described in detail.

The present specification proposes an apparatus which effectively inactivates viruses. The apparatus (1) includes one or more microwave antenna(s) (11) arranged for emitting a microwave frequency for inciting such mechanical damage in viruses. Said arrangement can be made using a designated (microwave) frequency for such purpose. Any embodiment of the apparatus (1) according to the present invention can include one or more of the following features: programmable analog / digital input outputs, one or more serial communication lines such as SPI (i.e. serial peripheral interface), RS232, RS485, USB, one or more processors, one or more RAM / ROM /FLASH Memories and may act as a SOM (system on module) or SOC (system on chip) or communication module for one or more external MCU(s) (microcontroller units), one or more graphical processor units. Such apparatus (1), in particular that arranged for acting as a SOM or communication module for one or more external MCU(s) can be thus considered as a "module". The apparatus (1) (e.g. such "module") can include a PCB (printed circuit board), or can be constituted from several other components provided on a PCB.

The apparatus makes use of acoustic vibration, thereby causing resonance which mechanically damages outmost layers of viruses. Although the apparatus is effective in a wide variety of viruses (such as various types of Ebola, influenza, SARS, etc.) by easily arranging the frequency and energy density levels in accordance with a respective virus; the inactivation of spherical envelops is particularly easy due to their geometric limitations in terms of envelope surface area capable of holding the genetic material and enzymes in their inner volume. Thus, the inactivation occurs instantly (i.e. within a very short residence time) and with consumption of a very low amount of energy. In particular, disintegration of envelopes requires a minimum amount of energy and time, in inactivation of lipid enveloped viruses such as coronaviruses. Yet, advantages of the apparatus are not to be limited only to those related to coronaviruses, and it is possible to inactivate other viruses and even microorganisms which require a higher extent of acoustic vibration in disintegration thereof.

Within the context of the present application, the apparatus (1) applies acoustic resonance to its surroundings.

Within the context of the present application, the term "microwave" (or "virus inactivating microwave") refers to that with a frequency to induce acoustic vibration on an outermost layer of viruses, causing mechanical damage of such outermost layer. It is observed that virus species can be destroyed in this way, by defining and selecting microwave frequency ranges specific thereto. For instance, it is observed that frequencies within the range between 8.0 GHz and 9.5 GHz cause momentary deformations on envelopes of SARS-COV-2, resulting in rupture of said envelopes. Therefore, this range is considered suitable for being employed in damaging coronaviruses. When the frequency is within the narrower range between 8.2 GHz and 8.8 GHz, the efficiency in inactivation of SARS-COV-2 further increases, and a frequency around 8.4 GHz (i.e. within the range between 8.3 GHz and 8.5 GHz) is considered sweet-spot in inactivation of such viruses in lowest time and with lowest energy consumption.

Since the present invention relies on mechanically damaging viruses by acoustic resonance, the apparatus (1) practically does not necessitate nor cause any heating of its surroundings, e.g. fluids or rigid items within an effective electromagnetic field of the apparatus (1). Considering that heating would denature further biochemical/biological structures other than viruses, the apparatus (1) according to the present invention also enables a safe and effective sterilization of liquids such as vaccines, or bodily fluids such as blood within an effective electromagnetic field of the apparatus (1). Therefore, the apparatus (1) according to the present invention and method of virus inactivation by using such apparatus is not to be limited for use in virus inactivation in air, water or rigid surfaces, but also to in-vitro or in-vivo sterilization of other fluids with delicate organic or biological material which should be maintained, such as vaccines, blood samples, spinal fluid samples, and even blood or spinal fluid of living organisms (e.g. mammals).

The apparatus (1) includes one or more microwave antenna(s) (11) arranged for emitting a microwave frequency for inciting such mechanical damage in viruses. Said arrangement can be made using a designated (microwave) frequency for such purpose.

The microwave with such designated frequency can be thus applied into the block (20) via said one or more microwave antenna(s) (11), thereby causing acoustic resonance on the outermost layer of viruses. For instance, in SARS-COV-2, the momentary envelope deformations acoustic resonance causes deviations from its original diameter (to extents calculable to be around 30 nm), resulting in inactivation due to mechanical damage in envelope structure. The same applies to other virus species by selection and easily replacing the one or more microwave antenna(s) (11) in accordance with another, frequency specific to another virus. Considering different sizes and outmost layer structures, the same principle applies to virus species other than SARS-COV-2, by employing respective specific frequencies selected from a range between 1 GHz and 20 GHz. Considering that currently SARS-COV-2 is the most urgent issue of the world, the frequency is preferably to be kept within the range between 8.0 GHz and 9.5 GHz, more preferably between 8.2 GHz and 8.8 GHz, even more preferably between 8.3 GHz and 8.5 GHz. Considering that commercially available and low cost waveguides (such as WR90) can be used as microwave emitting connectors (11), the frequency can be arranged to be within the range between 6.5 GHz and 13 GHz which provides a single-mode provision of adjustable frequency, and within the range between 7 GHz and 12 GHz for an even higher accuracy. In rod-shaped (i.e. substantially cylindrical) viruses, 5.5-7 GHz provides a peak in resonance, and 20-25 GHz, 33-37 GHz and 45-50 GHz are also effective in inactivation of such viruses. On the other hand, a frequency range within 6-10 GHz is considered as an intersection applicable to inactivation of both spherical viruses and rod-shaped viruses. Hence, the apparatus (1) can be easily adapted in accordance with a specific frequency designated to inactivation of a target virus.

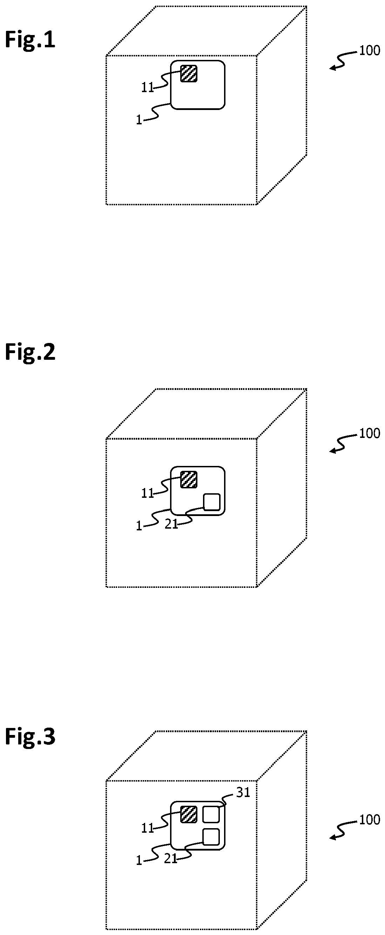

Fig.1 shows a schematic perspective view of an exemplary embodiment of the apparatus (1) according to the present invention, provided with an exemplary microwave antenna (11).

The apparatus (1) can further comprise a means for communication (21), and the apparatus (1) can be thus arranged for data exchange or interchange. Fig.2 shows an exemplary depiction of such embodiment. In this embodiment, the apparatus (1) has internet of things (IoT) compatibility. Such apparatus (1) can be considered as multifunctional. The means for communication (21) can be a means for internet connectivity. Such apparatus (1) can be further arranged to inherit any of the currently known further features related to IoT.

Preferably, the means for communication (21) can be arranged for triggering initiating or increasing power consumption of the one or more microwave antenna(s) (11). In such embodiment, the virus inactivation can be initiated or controlled using the means for communication (21). The arrangement of such triggering can be easily made using a suitable actuator.

For instance, this initiation can be performed remotely, e.g. using a remote computing device. Thereby, the virus inactivation can be started as a preparation prior to human approach into the vicinity of the apparatus (1); or the virus inactivation can be performed without necessitating an authorized operator to be physically present near the apparatus (1).

Preferably, the means for communication (21) and said microwave antenna(s) (11) can be formed as a single entity. In such embodiment, one or more microwave antenna(s) (11) are arranged for transmitting data embedded on the microwaves they generate/radiate. Thus, the means for communication (21) can be arranged to generate data signals embedded into the radiated microwaves used in virus inactivation. The means for communication (21) can be arranged such that said data signals are embedded on microwaves with any frequency range/value (and related characteristics) explicitly disclosed within the present detailed description directed to inactivate viruses. This embodiment of the apparatus (1) enables simultaneous performance of data communication and virus inactivation. The electric field achieved by said microwaves for virus inactivation can be received by a device which can receive said microwaves as data to be interpreted in the form of a signal; and communication is established along with virus inactivation. The means for communication (21) can have also IoT compatibility, e.g. by including a TCP/IP stack.

In any one of the embodiments mentioned in the present detailed description, the apparatus (1) can be arranged to allow increasing/decreasing momentary power consumption of the microwave antenna(s) (11) to boost/expedite virus inactivation when desired.

In any one of the embodiments, the apparatus (1) can be arranged for being brought into communication with one or more sensor(s) (31) such as an optical "presence" sensor e.g. a light dependent resistor (LDR), and/or a mechanical "presence" sensor e.g. a weight or pressure sensor, and/or a thermal "presence" sensor e.g. a temperature sensor; and such apparatus can comprise analog / digital connections (not shown) enabling such communication between the sensor and the apparatus (1); and the apparatus (1) can be pre-programmed to initiate and execute the virus inactivation in accordance with data/signals from the sensor(s) (31). Fig.3 shows an exemplary depiction of this embodiment. Such embodiment enables that the apparatus (1) can be autonomous (i.e. no real-time human intervention is required to initiate and execute virus inactivation), thereby the initiation and execution of virus inactivation can be automatized.

In any one of the embodiments, the means for communication (21) can be pre-programmed as a Doppler radar. Such embodiment can be arranged for normally reciprocate signal broadcast with a suitable, external receiver and function as a typical radar (over the amount of time past until signals echo from said receiver), and simultaneously perform virus inactivation.

### EXAMPLE 1: FIXTURE (100) WITH AN APPARATUS INTEGRATED THERETO:

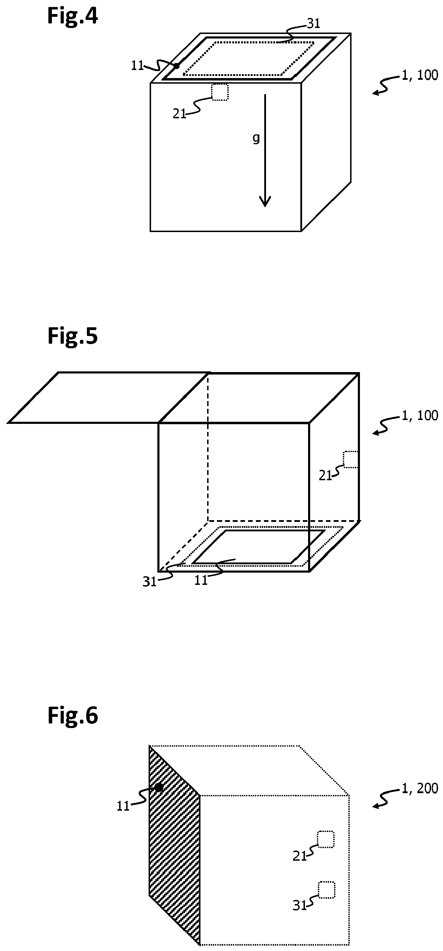

The apparatus (1) can be integrated as a part of a fixture (100) typically used in offices, houses/apartments, transportation vehicles, or public transportation vehicles such as buses, airplanes, vessels etc. In this case, it is possible to consider the fixture (100) as the apparatus (1) itself. An exemplary depiction of this embodiment is shown in Fig.4.

### EXAMPLE 1.1: IN FIXTURES (100) OF TRANSPORTATION VEHICLES

For instance, the apparatus (1) can be integrated as a part of a fixture (100) in/ or for use in transportation vehicles (e.g. bus, airplane, vessel, private car, taxi cab), preferably along with one or more sensor(s) (31) (e.g. presence sensors as mentioned above). For instance, the fixture (100) can be in the form of a seat. In such exemplary case, the seat can be designed and produced in accordance with known requirements/limitations/standards related to respective specific type of public transportation vehicle fixture (100), e.g. as a bus seat, or airplane seat, vessel seat, car seat, etc. Such embodiment can be arranged for broadcasting an increased extent of microwave energy (by increasing the power consumption of the microwave antenna (11) when the seat is not occupied. For instance, the presence sensor(s) (31) can be one or more pressure sensors to determine whether a body weight is present on the seat: the apparatus (1) can be arranged, at such instance of seat occupation, to cease or decrease energy consumption in microwave broadcasting. Such embodiment can be further arranged to initiate or increase energy consumption in microwave broadcasting when the seat is not occupied. Furthermore, the apparatus (1) can be provided with one or more further sensors for obtaining one or more information when the seat is occupied: the information can include for instance weight of the passenger, body temperature of the passenger, or humidity of ambient air. The apparatus (1) can be further arranged for generating an audible and/or visible signal as a notification related to that a virus inactivation session is in progress, and/or related to that a virus inactivation session is completed. In the latter case, a passenger can be ensured that it is completely safe to take seat. The transportation vehicle as an intended place for use of the fixture (100) integrated with the apparatus (1) can be preferably a public transformation vehicle, and more preferably an airplane fixture, such as an airplane seat or an airplane toilet seat. In the possible case where the intended place for use of the fixture (100) is a private car, taxi cab or bus, the fixture (100) can be in the form of a rear-view mirror or dashboard for use in the transportation vehicle.

The apparatus (1) can be arranged to generate and transfer data related to said information to a further device (e.g. a coordinating means or coordinator device to coordinate virus inactivation in the public transportation vehicle), via a mesh network (e.g. by including a modem) or via broadcasting by embedding the data onto said microwaves. In such case, it can be considered that the apparatus (1) provides an IoT application/compatibility. By arranging the type, size and shape of e.g. pressure sensors as presence sensor(s) (31), such apparatus (1) can be (or adapted to be) integrated to one or more of public transportation vehicle fixtures (100) such as seat handle, arm rest, bars, grab handles, and toilet seats within the same context (i.e. to initiate or increase power consumption of microwave antenna(s) (11), when the fixture (100) is momentarily not in use). Since the apparatus (1) can be produced with a minimal extent of weight, operated with a relatively low extent of power consumption, and expandable virus inactivation coverage area (e.g. by momentarily increasing power consumption of the microwave antenna(s) (11)); it is highly suitable for being integrated such fixtures (100) of airplanes.

In such applications, the microwaves generated/radiated/broadcast by the can be subjected to e.g. electrical screening and captured into Faraday's cage in order to prevent data signal pollution to other, irrelevant communication systems in a respective public transportation vehicle.

### EXAMPLE 1.2: IN HOME/APARTMENT OR OFFICE FIXTURES (100)

In a possible application, the apparatus (1) according to the present invention can be integrated to a household or office fixture (100), such as furniture. The apparatus (1) can be battery powered and/or arranged to be connected to mains power as a standard power source. The apparatus (1) can be arranged for being brought into communication with one or more sensor(s) (31) (not shown) such as an optical "presence" sensor e.g. a light dependent resistor (LDR), and/or a mechanical "presence" sensor e.g. a weight or pressure sensor, and/or a thermal "presence" sensor e.g. a temperature sensor; and analog/digital connections enabling such communication between the sensor and the apparatus (1); and the apparatus (1) can be pre-programmed to initiate and execute the virus inactivation in accordance with data/signals from the sensor(s) (31). Such embodiment enables that the apparatus (1) can be autonomous (i.e. no real-time human intervention is required to initiate and execute virus inactivation), thereby the initiation and execution of virus inactivation can be automatized.

In particular, said one or more sensor(s) (31) can include a pressure sensor, e.g. provided on the top surface of the fixture (100), for detecting a change in values of weight exerted onto the top surface when the fixture (100) is in use. Said one or more sensors can further include a temperature sensor, e.g. provided on the top surface of the furniture, for detecting thermal values on the top surface. The apparatus (1) can be arranged to generate and transfer data related to information based on one or more of said values to a further device (e.g. a coordinating means or coordinator device to coordinate virus inactivation in a facility where the fixture (100) is placed, e.g. a restaurant including a desk as a fixture (100)), via a mesh network (e.g. by including a modem) or via broadcasting by embedding the data onto said microwaves. In such case, it can be considered that the apparatus (1) provides an IoT application/compatibility.

Within this context, the fixture (100) can be a desk having a top surface provided with the apparatus (1), such that the top surface includes one or more microwave antenna(s) (11). For instance, when food items are served by being placed onto the top surface, said one or more sensor(s) (31) detect the presence of the served food items, and then, virus inactivation on the food items can be automatically triggered by initiating or increasing the power consumption of the microwave antenna(s) (11). The temperature sensor can also provide information on ambient temperature or food temperature based on momentary top surface temperature. Such apparatus (1) can be in communication with a user interface (such as a touch screen) for enabling communication of user options (such as orders to be taken from a customer as a user) to the above-mentioned further device.

Similarly, the fixture (100) can be a seat, a chair, a sofa, a bed, a carpet, rug or prayer rug etc., having a top surface provided with the apparatus (1), such that the top surface includes one or more microwave antenna(s) (11). For instance, when a user at least partly gives his weight onto the top surface to use the fixture (100), and eventually the weight is removed from the top surface, said one or more sensor(s) (31) detect that the user ceases using the fixture (100), and virus inactivation at the top surface can be automatically triggered by initiating or increasing the power consumption of the microwave antenna(s) (11). The temperature sensor can also provide information on body temperature of the user based on momentary top surface temperature.

Furthermore, the fixture (100) provided with the apparatus (1) can be a lighting fixture (100) such as a floor lamp, table lamp or chandelier. Such apparatus (1) can be arranged for initiating the virus inactivation based on a user command. Preferably, the apparatus (1) can be arranged for automatically initiating or ceasing the virus inactivation based on a presence sensor data: for instance, the virus inactivation can be initiated when it is detected that nobody is present in the covering range of virus inactivating microwaves of the microwave antenna (1); and/or the virus inactivation can be ceased when it is detected that a person or pet approaches to the covering range of virus inactivating microwaves of the microwave antenna (1).

The fixture (100) with an integrated apparatus (1) within the above context can be for use in public buildings; such as waiting room seats, counters, lighting fixtures (100), and in particular elevators. The virus inactivating behaviour of the apparatus (1) is especially useful in public building fixtures (100) and particularly, elevators. This is because the number of different persons using the fixture (100) is expected to be very high when considered per hour.

The fixture (100) can be a box provided with the apparatus (1) according to the present invention, such that the one or more microwave antenna(s) (1) are arranged for virus inactivation directed to an inner cavity of the box. Fig.5 shows an exemplary depiction of such apparatus. For instance; goods or bags brought from shopping can be placed into the box, and then the virus inactivation can be triggered/initiated based on a user command, or preferably automatically. Such automatic triggering/initiation can be based on detection of that a lid of the box is closed. Detection of that the box is closed can be based a signal or data generated by one or more of the following sensors: one or more position sensor(s) (31) for detecting that the lid is brought into a closed state; one or more light sensor(s) (31) for detecting that the light received into the inner cavity of the box decreases; and/or one or more pressure sensor(s) (31) for detecting weight of goods when placed into the inner cavity.

### EXAMPLE 2: IN HARD GOODS SUCH AS SMALL (HOME) APPLIANCES OR WHITE GOODS

In a possible application, the apparatus (1) according to the present invention can be integrated to a hard good (200) such as a small appliance (e.g. toaster, coffee machine, etc.) or a white good (e.g. refrigerator, dishwasher, laundry/washing machine). The apparatus (1) can be battery powered and/or arranged to be connected to mains power as a standard power source. The apparatus (1) can be arranged for being brought into communication with one or more sensor(s) (31) (not shown) such as an optical "presence" sensor e.g. a light dependent resistor (LDR), and/or a mechanical "presence" sensor e.g. a weight or pressure sensor, and/or a thermal "presence" sensor e.g. a temperature sensor; and analog/digital connections enabling such communication between the sensor and the apparatus (1); and the apparatus (1) can be pre-programmed to initiate and execute the virus inactivation in accordance with data/signals from the sensor(s) (31). Such embodiment enables that the apparatus (1) can be autonomous (i.e. no real-time human intervention is required to initiate and execute virus inactivation), thereby the initiation and execution of virus inactivation can be automatized. It is possible to consider the hard good (200) as the apparatus (1) itself.

The apparatus (1) can be arranged to generate and transfer data related to information based on one or more of values generated by the one or more sensor(s) (31) to a further device (e.g. a coordinating means or coordinator device to coordinate virus inactivation in a facility where the hard good (200) is placed, e.g. a kitchen or bathroom including the hard good), via a mesh network (e.g. by including a modem) or via broadcasting by embedding the data onto said microwaves. In such case, it can be considered that the apparatus (1) provides an IoT application/compatibility: for instance, any parameter of e.g. a refrigerator can be remotely followed.

The apparatus (1) can be arranged for enable virus inactivation of a room (e.g. kitchen, bathroom, etc.) by providing an expansive covering area (or volume) of virus inactivating microwaves, e.g. size, shape and power consumption rates of one or more microwave antenna(s) can be arranged for covering a volume of at least 27 m³ (e.g. 3m x 3m x 3m, to cover a bathroom or a kitchen) under an extent of electrical field suitable for virus inactivation.

Within this context, the hard good (200) can be a white good (refrigerator, dishwasher, laundry drier, washing machine, etc.) having a side surface (shell) provided with the apparatus (1), such that the side surface includes one or more microwave antenna(s) (11). In a possible embodiment, the side surface (e.g. body shell) can function as a microwave antenna (11) as a whole. This measure can also apply to small appliances as exemplified above. Fig.6 shows an exemplary depiction of a small appliance or white good provided with the apparatus (1), wherein a side surface is arranged to function as a microwave antenna (11) to broadcast virus inactivating microwaves.

### EXAMPLE 3: IN CONVEYORS (e.g. BELT CONVEYORS)

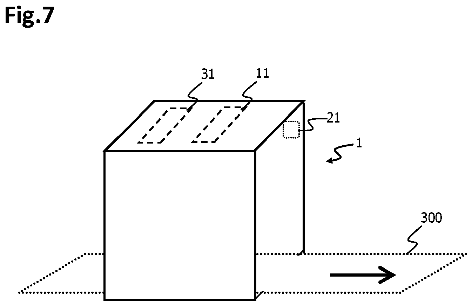

In a possible application, the apparatus (1) according to the present invention can be integrated to a conveyor (300), such that the one or more microwave antenna(s) (11) are arranged for covering goods (e.g. packages, supermarket products, service plates to support catering products, kitchen artillery, biological samples, vaccines) to be conveyed by said conveyor (300). Hence, the goods can be subjected to virus inactivation whilst being conveyed. Fig.7 shows an exemplary version of the apparatus (1) according to the present invention integrated with an exemplary conveyor (300). For instance, in the case of belt conveyor (300) for supporting goods against gravity, the microwave antenna(s) (11) can be positioned such that an upper surface of the belt conveyor (300) is covered by the microwave antenna(s) (11).

The apparatus (1) can be arranged for adapting the power consumption of the microwave antenna(s) (11) in accordance with conveyor (300) speed; such that an increased extent of power consumption (for rapid virus inactivation) is applied at higher conveyor (300) speeds.

The apparatus (1) can be provided with one or more "presence" sensor(s) (31) as exemplified above, to detect momentary coordinates/positions of goods approaching/entering to the coverage area of the microwave antenna(s) (11). The apparatus (1) can be arranged to increase the power consumption (or initiate the power consumption) of the microwave antenna(s) (11) at instances when goods are conveyed through said coverage area. Alternatively, or furthermore, the apparatus (1) can be arranged to focus/direct the virus inactivating microwaves towards the goods conveyed through the coverage area.

A plurality of such apparatuses (1) can be provided along a single conveyor, and can be arranged for consecutively exerting virus inactivation onto a conveyed good, such that the virus inactivation of a conveyed good is completed upon passing through respective covering areas of microwave antenna(s) (11) of a plurality of apparatuses (1).

The apparatus (1) can be arranged to generate and transfer data related to information based on one or more of values generated by said one or more sensor(s) (31) to a further device (e.g. a coordinating means or coordinator device to coordinate virus inactivation in a facility where the hard good (200) (200) is placed, e.g. a kitchen or bathroom including the hard good), via a mesh network (e.g. by including a modem) or via broadcasting by embedding the data onto said microwaves. In such case, it can be considered that the apparatus (1) provides an IoT application/compatibility: for instance, any parameter of e.g. a refrigerator can be remotely followed.

The arrangement of power consumption or direction of microwave antenna(s) (11), and/or the conveying speed/status can be enabled using a coordinating means having a processor and algorithm (e.g. for controlling the speed or status of the conveyor (300) over a motor of said conveyor (300)). Said coordinating means can be in communication with one or more of the apparatus (1) integrated to a conveyor. An above-mentioned plurality of apparatus (1) can be provided with electronic cards arranged as a "distributed" coordinating means working together. Data related to the conveyor, can be communicated to a further device (e.g. a coordinating means remotely positioned from the conveyor (300)) by one or more means for communication (21) provided at one or more apparatus (1) integrated to a conveyor. Said data related to the conveyor (300) can include: conveying status (e.g. "stopped" or "running"), conveying speed (e.g. in meters per second), presence of the goods, density of the goods (e.g. items subjected to microwaves, per minute), virus inactivation status (e.g. virus inactivation "in progress" or "completed").

### EXAMPLE 4: IN WEARABLE DEVICES

In a possible application, the apparatus (1) according to the present invention can be integrated to a wearable technological device. The wearable device can be exemplified as a smart wrist band, a smart watch, smart shoes, smart facemask, smart shirt, smart jacket, etc.

### EXAMPLE 5: IN BATTERY-POWERED PORTABLE (e.g. POCKET-SIZE OR HAND-HELD) DEVICES

In a possible application, the apparatus (1) according to the present invention can be integrated to a portable device, for user-directed virus inactivation. The portable device can be a pocket-size, or hand-held device. For instance, the portable device can be a battery-powered hand lamp, or e.g. a pen-shaped device for virus inactivation when in use. In a preferred embodiment, the portable device can be a communication device such as mobile phone, which can be further arranged for generating communication signals embedded on virus inactivating microwaves. This enables a high-speed communication of data such as video and audio transfer at video calls.

### EXAMPLE 6: IN CONSUMER ELECTRONICS AND OTHER ELECTRONIC DEVICES

In a possible application, the apparatus (1) according to the present invention can be integrated to a portable computer, such as a laptop or tablet pc. Considering the possible and widely accepted use of portable computers in wireless communication, the portable computer can be further arranged for generating communication signals embedded on virus inactivating microwaves. This enables a high-speed communication of data such as video and audio transfer at video calls.

In another possible application, the apparatus (1) according to the present invention can be integrated to a modem, preferably Wi-Fi modem. Preferably, the modem is arranged for embedding data onto microwaves (e.g. virus inactivating microwaves). Such embodiment is even more preferable since it allows to constitute a high-speed network for communication of data with a mobile phone mentioned in the Example 5, which is arranged for generating communication signals embedded on virus inactivating microwaves.

In another possible application, the apparatus (1) according to the present invention can be integrated to an electronic security system with communication abilities; thereby providing virus inactivation ability to such security system.

In another possible application, the apparatus (1) according to the present invention can be integrated to an energy management and control system, arranged to communicate with one or more sensors (such as the above-mentioned one or more "presence" sensors, and/or ambient temperature sensors) and thus constitutes a communication backbone. Such system can be arranged for virus inactivation at a location in which the apparatus (1) is placed (e.g. at a thermostat).

In another possible application, the apparatus (1) according to the present invention can be integrated to a smart loudspeaker. The smart loudspeaker can be arranged to communicate with one or more sensors (such as the above-mentioned one or more "presence" sensors, and/or ambient temperature sensors), microphones and one or more further smart loudspeakers; thereby enabling virus inactivation simultaneous to data communication (e.g. by embedding data signals onto virus inactivating microwaves).

### Reference signs:

- 1: apparatus
- 11: microwave antenna(s)
- 21: means for communication
- 31: sensor
- 100: fixture
- 200: hard good
- 300: conveyor

## Claims

1. An apparatus (1) which includes one or more microwave antenna(s) (11) arranged for emitting a microwave frequency for inciting such mechanical damage in viruses.

2. The apparatus (1) according to the claim 1, wherein said one or more microwave antenna(s) (11) are arranged for emitting a frequency within the range between 1 GHz and 20 GHz.

3. The apparatus (1) according to the claim 2, wherein said one or more microwave antenna(s) (11) are arranged for emitting a frequency within the range between 8.0 GHz and 9.5 GHz, more preferably between 8.2 GHz and 8.8 GHz, even more preferably between 8.3 GHz and 8.5 GHz.

4. The apparatus (1) according to any of the claims 1 to 3, further comprising one or more means for communication (21).

5. The apparatus (1) according to the claim 4, wherein the means for communication (21) is arranged for triggering an initiation or an increment of a power consumption of the one or more microwave antenna(s) (11).

6. The apparatus (1) according to any of the claims 1 to 5, arranged for being brought into communication with one or more sensor(s) (31); and the apparatus (1) comprises analog/digital connections for the arrangement of the communication between the sensor(s) (31) and the apparatus (1); and the apparatus (1) is pre-programmed to initiate and execute the virus inactivation in accordance with data/signals from the sensor(s) (31).

7. The apparatus (1) according to any of the claims 1 to 6, integrated as a part of a fixture (100) selected from seat handle, arm rest, bar, grab handle, lighting fixture and toilet seat.

8. The apparatus (1) according to the claim 6, wherein the apparatus is integrated as a part of a fixture (100) selected from seat handle, arm rest, bar, grab handle, lighting fixture and toilet seat, said one or more sensor(s) being arranged to generate a value related to whether the fixture (100) is in use, and the apparatus (1) is arranged to generate and transfer data related to information based on one or more of said values to a coordinating means for coordination of virus inactivation in a facility where the fixture (100) is placed via a mesh network or via broadcasting by embedding the data onto microwaves generated by the microwave antenna(s) (11); and the fixture (100) is preferably a public transportation vehicle fixture.

9. The apparatus (1) according to any of the claims 1 to 6, integrated as a part of a fixture (100) which is in the form of a box, such that the one or more microwave antenna(s) (1) are arranged for virus inactivation directed to an inner cavity of the box.

10. The apparatus (1) according to any of the claims 1 to 6, integrated as a part of a small home appliance or white good, wherein the one or more microwave antenna(s) (11) are preferably in the form of an outer shell of said home appliance or white good.

11. The apparatus (1) according to any of the claims 1 to 6, integrated to a conveyor (300), such that the one or more microwave antenna(s) (11) are arranged for covering goods to be conveyed by said conveyor (300).

12. The apparatus (1) according to any of the claims 1 to 6, integrated to a portable hand-held device, which is preferably a mobile phone, which is even more preferably further arranged for generating communication signals embedded on the microwaves.

13. The apparatus (1) according to any of the claims 1 to 6, integrated to an elevator.

14. The apparatus (1) according to the claim 6, integrated as a part of a fixture (100), and said one or more sensor(s) (31) including one or more pressure sensor(s) for detecting a change in values of weight exerted onto a top surface of the fixture (100) when the fixture (100) is in use; and/or a temperature sensor for detecting thermal values on the top surface; more preferably the apparatus (1) is arranged to generate and transfer data related to information based on one or more of said values to a coordinating means for coordination of virus inactivation in a facility where the fixture (100) is placed via a mesh network or via broadcasting by embedding the data onto microwaves generated by the microwave antenna(s) (11).

15. The apparatus (1) according to the claim 14, wherein the fixture (100) is selected from seat, chair, sofa, bed, desk, carpet, rug and toilet seat.
